# EUROPÄISCHE PATENTSCHRIFT

(11) **EP 2 411 801 B1**
(45) Veröffentlichungstag und Bekanntmachung des Hinweises auf die Patenterteilung: **07.11.2012**
(21) Anmeldenummer: 10708770.2
(22) Anmeldetag: 15.03.2010
(51) Int. Cl.: G01N 33/18, G01N 35/10

(54) **VERFAHREN ZUM BESTIMMEN EINER ANZAHL VON TROPFEN**
METHOD FOR DETERMINING A NUMBER OF DROPS
PROCÉDÉ PERMETTANT DE DÉTERMINER UN NOMBRE DE GOUTTES

(30) Priorität: 25.03.2009 DE 102009001860
(43) Veröffentlichungstag der Anmeldung: 01.02.2012
(73) Patentinhaber: Endress+Hauser Conducta Gesellschaft für Mess- und Regeltechnik mbH & Co. KG, 70839 Gerlingen (DE)
(72) Erfinder: Bettmann, Oliver, 65428 Rüsselsheim (DE); Kathe, Ulrich, 71640 Ludwigsburg (DE)
(74) Vertreter: Andres, Angelika Maria
(86) Internationale Anmeldenummer: PCT/EP2010/053266
(87) Internationale Veröffentlichungsnummer: WO 2010/108801

(56) Entgegenhaltungen:
- DE-A1- 10 127 353
- DE-A1- 19 617 910
- DE-A1-102006 058 051
- DE-C1- 4 344 441

## Beschreibung

Die vorliegende Erfindung betrifft ein Verfahren zum Bestimmen einer Anzahl von Tropfen, welche mit einer Tropffrequenz in ein Reaktionsgefäß, insbesondere in einer Hochtemperaturaufschluss-einrichtung für Analysatoren, eindosiert werden.

Das tropfenweise Eindosieren von Flüssigkeiten in ein Reaktionsgefäß spielt beispielsweise in der Analysetechnik eine Rolle. Häufig sind in solchen Anwendungen Analyten oder Reagenzien zu dosieren. Das tropfenweise Dosieren von Flüssigkeiten soll allgemein den Zweck erfüllen, ein definiertes Volumen einer Substanz in das Reaktionsgefäß einzubringen.

Eine beispielsweise in der Abwasseranalyse wichtige Anwendung, bei der der der Analyt tropfenweise in ein Reaktionsgefäß einzudosieren ist, ist die Bestimmung des Kohlenstoffgehalts und/oder des Stickstoffgehalts in Abwasser, beispielsweise des TOC (Total Organic Carbon, gesamter organisch gebundener Kohlenstoff) oder des TN_{b} (Total Nitrogen, gesamter gebundener Stickstoff). Bei einem aus DE102006058051 bekannten Verfahren zur Bestimmung dieser Parameter wird eine Flüssigkeitsprobe von einem geringen Volumen von beispielsweise einigen 100 µl tropfenweise einem Reaktionsgefäß der Hochtemperaturaufschlußeinrichtung zugeführt. Im Reaktionsgefäß, das beispielsweise durch einen als Pyrolyserohr ausgebildeten Hochtemperaturreaktor gebildet wird, werden die organischen Inhaltsstoffe thermisch zu CO₂, die Stickstoff enthaltenden Inhaltsstoffe zu Stickstoffoxid NOₓ aufgeschlossen. Die Kurzschreibweise NOₓ steht hier für ein Gemisch aus Stickoxiden mit Stickstoff in verschiedenen Oxidationsstufen, das jedoch als Hauptkomponente NO aufweist. Bei der Umsetzung im Hochtemperaturreaktor entsteht ein Gasgemisch, das neben CO₂ und NOₓ gasförmiges H₂O und gegebenenfalls weitere Pyrolyse- und Reaktionsprodukte von in der Probe enthaltene Substanzen enthält. Das Gasgemisch wird mit Hilfe eines permanent das Reaktionsgefäß durchströmenden Trägergases, das in der Regel auch den nötigen Reaktionssauerstoff liefert, durch einen Kühler mit Wasserabscheider, einen Gasfilter und eine Analyseeinheit transportiert. Die Menge des entstandenen CO₂ bzw. NOₓ wird, beispielsweise durch Infrarotmessung oder durch Chemilumineszenzmessung, bestimmt, und aus diesem Wert der TOC- bzw. TN_{b}-Gehalt der Flüssigkeitsprobe bestimmt.

Die in der Hochtemperaturaufschlusseinrichtung herrschenden Temperaturen liegen im Betrieb deutlich über dem Siedepunkt der eindosierten flüssigen Probe. Bei der TOC- oder TN_{b}-Bestimmung herrscht im Inneren des Reaktionsgefäßes üblicherweise eine Temperatur von ca. 650°C bis zu 1300°C, je nachdem, ob das Aufschließen der Probe zusätzlich durch einen Katalysator unterstützt wird. In Kontakt mit der Wand des Reaktionsgefäßes oder sonstiger innerhalb des Reaktionsgefäßes vorliegender Oberflächen erreicht ein Flüssigkeitstropfen innerhalb kürzester Zeit Siedetemperatur bzw. die zur Reaktion mit dem im Trägergas enthaltenen Sauerstoff erforderliche Reaktionstemperatur. Ein in das Reaktionsgefäß eindosierter Flüssigkeitstropfen geht daher unmittelbar nach dem Eindosieren in das Reaktionsgefäß durch Verdampfen und/oder durch Bildung gasförmiger Reaktionsprodukte in die Gasphase über.

Bei der Bestimmung der Analytkonzentration, beispielsweise des TOC- oder TN_{b}-Werts, kommt es darauf an, das Volumen der in das Reaktionsgefäß eindosierten Probeflüssigkeit genau zu kennen. Insbesondere bei kleinen Probenvolumina im µl- bzw. unteren ml-Bereich, können sich bei Fehldosierungen nicht tolerierbare Abweichungen der ermittelten Analytkonzentration von der tatsächlich vorliegenden Analytkonzentration ergeben. Geht man beispielsweise davon aus, dass ein Volumen von 400 µl einer wässrigen Lösung etwa einer Anzahl von 20 Tropfen entspricht, so ergibt sich bei einer Fehldosierung, bei der nur 19 Tropfen in das Reaktionsgefäß eindosiert werden, bereits eine Abweichung des tatsächlich eindosierten Probenvolumens vom vorgegebenen Probenvolumen um 5%.

Fehldosierungen können jedoch gerade im automatisierten Betrieb einer Analysevorrichtung immer wieder auftreten, beispielsweise durch Zusetzen der Probenzuleitung durch in der Probe enthaltene feste Partikel oder aufgrund von Gasblasen in der einzudosierenden Probe. Es ist daher zur Gewährleistung eines korrekten Analyseergebnisses wünschenswert, das Eindosieren der Probe in das Reaktionsgefäß zu überwachen.

In der Infusionstechnik, beispielsweise aus EP 237773 A1 ist es bekannt, Tropfen durch optische Mittel, beispielsweise durch Lichtschranken, zu erkennen, und auf diese Weise zu überwachen, wie viele Tropfen einer Flüssigkeit tatsächlich dosiert werden. Eine derartige Vorrichtung ist jedoch apparativ aufwändig, und insbesondere im Hinblick auf die beschriebenen Anwendungen im Hochtemperaturbereich nur schwer zu realisieren. Selbst wenn das Reaktionsgefäß der Hochtemperaturaufschlusseinrichtung transparent, beispielsweise aus Quarzglas gebildet wäre, und die Lichtschranke außerhalb des Pyrolyserohrs angeordnet würde, wäre eine derartige Überwachung der Eindosierung von Tropfen in das Pyrolyserohr nicht praktikabel, da die Transparenz des Quarzglases über die Betriebsdauer durch Niederschlag von Salzen und durch lokale Kristallisation der Quarzglasmatrix durch Einfluss von Alkalimetallionen unter hohen Temperaturen abnimmt.

Es ist daher Aufgabe der Erfindung, ein Verfahren zum Bestimmen einer Anzahl von Tropfen einer Flüssigkeit, die in ein Reaktionsgefäß eindosiert werden, anzugeben, das die Nachteile des Standes der Technik überwindet. Insbesondere soll ein Verfahren angegeben werden, mit dem sich die Anzahl von Tropfen einer Flüssigkeit, die in ein Reaktionsgefäß eindosiert werden, mit hoher Genauigkeit bestimmen lässt, und welches insbesondere für Anwendungen geeignet ist, bei denen im Reaktionsgefäß eine Temperatur herrscht, welche höher ist als die Siedetemperatur der Flüssigkeit.

Die Aufgabe wird gelöst durch ein Verfahren zum Bestimmen einer Anzahl von Tropfen, welche mit einer Tropffrequenz in ein Reaktionsgefäß, insbesondere in einer Hochtemperaturaufschlusseinrichtung für Analysatoren, eindosiert werden,
wobei das Reaktionsgefäß von einem Gasstrom durchströmt wird,
und wobei im Reaktionsgefäß eine Temperatur herrscht, welche größer ist als die Siedetemperatur der Flüssigkeit,
und wobei ein in das Reaktionsgefäß eindosierter Tropfen, insbesondere durch Wärmeübertragung in Kontakt mit einer Oberfläche innerhalb des Reaktionsgefäßes, nach dem Eintritt in das Reaktionsgefäß, insbesondere unmittelbar nach Kontakt mit der Oberfläche innerhalb des Reaktionsgefäßes, mindestens teilweise, insbesondere vollständig in die Gasphase übergeht,
wobei mit einer Abtastrate, welche größer ist als die Tropffrequenz eine Folge von vom Druck innerhalb des Reaktionsgefäßes abhängigen Drucksignalen erfasst wird, und aus der Folge von Drucksignalen oder daraus abgeleiteten Werten eine Anzahl von in das Reaktionsgefäß eindosierten Tropfen ermittelt wird.

Da die Temperatur des Reaktionsgefäßes über der Siedetemperatur der eindosierten Flüssigkeit liegt, geht ein Tropfen dieser Flüssigkeit unmittelbar nach Eintritt in das Reaktionsgefäß durch Verdampfen und/oder durch Bildung gasförmiger Reaktionsprodukte in die Gasphase über. Insbesondere bei Kontakt mit einer Oberfläche innerhalb des Reaktionsgefäßes; beispielsweise der Innenwand des Reaktionsgefäßes oder einer Oberfläche eines im Reaktionsgefäß angeordneten Einsatzes, erfolgt der Wärmeübergang auf den Tropfen besonders schnell, z.B. innerhalb weniger als 0,3 s, insbesondere innerhalb weniger als 0,1 s, so dass der Tropfen unmittelbar nach Kontakt mit der Oberfläche in die Gasphase übergeht. Innerhalb des Reaktionsgefäßes kann ein Einsatz vorgesehen sein, der Schüttgut enthält, an dessen Oberfläche ein derartiger schneller Wärmeübergang auf auftreffende Tropfen erfolgen kann. Der Übergang des Tropfens in die Gasphase führt zu einer kurzfristigen Erhöhung des Drucks, im Folgenden als Druckstoß oder Druckimpuls bezeichnet, innerhalb des Reaktionsgefäßes. Indem mit einer Abtastrate, welche höher ist als die Tropffrequenz, d.h. der Anzahl von in das Reaktionsgefäß eindosierten Tropfen pro Zeiteinheit, eine Folge von Drucksignalen erfasst wird, ist gewährleistet, dass sich jeder durch einen Tropfen bewirkte Druckimpuls in der erfassten Folge von Drucksignalen widerspiegelt. Auf diese Weise kann also die Tropfenzahl mit hoher Genauigkeit bestimmt werden, und eine Abweichung zwischen der vorgegebenen, einzudosierenden Tropfenzahl und der tatsächlichen Anzahl der eindosierten Tropfen festgestellt werden.

Die Drucksignale können mittels eines Druckmesswandlers erfasst werden, der bevorzugt innerhalb des Gasstroms angeordnet ist. Unter einem innerhalb des Gasstroms angeordneten Druckmesswandler ist ein Druckmesswandler zu verstehen, der an einer beliebigen Position entlang des Strömungsweges des Gasstroms angeordnet ist. Vorzugsweise ist diese Position außerhalb des Reaktionsgefäßes gewählt, da dort niedrigere Temperaturen herrschen als innerhalb des Reaktionsgefäßes. Strömungswiderstände im Verlauf des Gasstroms führen dazu, dass Druckänderungen innerhalb des Reaktionsgefäßes auch von einem außerhalb des Reaktionsgefäßes innerhalb des Gasstroms angeordneten Druckmesswandler detektierbar sind, beispielsweise von einem innerhalb einer Zuführung für den Gasstrom in das Reaktionsgefäß angeordneten Druckmesswandler.

Zur Ermittlung der Anzahl der in das Reaktionsgefäß eindosierten Tropfen kann aus der Folge von Drucksignalen ein aktuelles Drucksignal (Pₙ) durch Vergleich mit einem Basisdruckwert (Pₘᵢₜₜₑₗ) eine zum aktuellen Drucksignal (Pₙ) zugehörige Druckänderung (P_{delta}) ermittelt werden, und die Druckänderung (P_{delta}) mit einem vorgegebenen Schwellenwert verglichen werden, und auf Grundlage eines Ergebnisses des Vergleichs erfasst werden, ob die Druckänderung (P_{delta}) einem durch Eindosieren eines Tropfens in das Reaktionsgefäß bewirkten Druckstoß entspricht.

Dabei kann der Basisdruckwert (Pₘᵢₜₜₑₗ) unter Verwendung von mindestens zwei in der Folge von Drucksignalen dem aktuellen Drucksignal (Pₙ) vorangehenden Drucksignalen durch Mittelwertbildung, insbesondere durch gleitende Mittelwertbildung, gebildet werden. Beispielsweise kann der Basisdruckwert zu Beginn des Verfahrens auf den vor Beginn der Probeneindosierung in dem Reaktionsgefäß herrschenden Druck gesetzt werden. Während des Eindosierens der Flüssigkeit werden aufeinander folgende Drucksignale erfasst, und der Basisdruckwert durch gleitende Mittelwertbildung unter Einbeziehung der jeweils aktuellsten Drucksignale der Folge nachgeführt.

Überschreitet die Druckänderung (P_{delta}) den vorgegebenen Schwellenwert, kann das Eindosieren eines Tropfens in das Reaktionsgefäß registriert werden. Zum Registrieren eines Tropfens kann eine, beispielsweise in einem Zähler, hinterlegte Anzahl von in das Reaktionsgefäß eindosierten Tropfen im Betrag um den Wert eins erhöht werden.

Um zu vermeiden, dass der Basisdruckwert Pₘᵢₜₜₑₗ zu hoch angesetzt wird, kann es von Vorteil sein, bei Überschreiten des vorgegebenen Schwellenwerts das zu der entsprechenden schwellenwert-überschreitenden Druckänderung (P_{delta}) gehörige aktuelle Drucksignal (Pₙ) nicht zur Berechnung des Basis-Druckwertes (Pₘᵢₜₜₑₗ) zu verwenden. Da der Basisdruckwert Pₘᵢₜₜₑₗ im Wesentlichen dem im Reaktionsgefäß herrschenden "Hintergrunddruck", der ungeachtet der Tropfenereignisse vorliegt, entspricht, stellt er die "Nulllinie" oder "Basislinie" des Druckverlaufs dar. Das Einbeziehen der durch den aufgrund des Tropfens entstehenden Druckimpuls erhöhten Drucksignale würde deshalb zu einem zu hohen Basisdruckwert führen. Nicht zu berücksichtigende Drucksignale der Folge können beispielsweise mittels eines Tiefpassfilters bei der Berechnung des Basisdruckwertes Pₘᵢₜₜₑₗ eliminiert werden.

Um weiterhin zu vermeiden, dass ein Druckimpuls mehrfach gezählt wird, kann es vorteilhaft sein, auf einen zu einer Druckänderung (P_{delta}), die einem durch Eindosieren eines Tropfens in das Reaktionsgefäß bewirkten Druckstoß zugeordnet wird, gehörenden Drucksignals (Pₙ) folgende, innerhalb eines vorgegebenen Zeitfensters erfasste Drucksignale bei der Bestimmung der Tropfenzahl nicht zu berücksichtigen. Eine für auf das aktuelle Drucksignal Pₙ folgende Drucksignale ermittelte Schwellenwertüberschreitung soll somit nicht zu einer erneuten Registrierung des Eindosierens eines Tropfens führen. Dabei ist es vorteilhaft das besagte Zeitfenster so breit zu wählen, dass die zum ersten, nach Ablauf des Zeitfensters erfassten aktuellen Drucksignal gehörige Druckänderung den vorgegebenen Schwellenwert unterschreitet.

Dies kann beispielsweise bewirkt werden, indem der Zähler durch eine Steuerung für die Dauer des Zeitfensters deaktiviert wird. Alternativ kann eine Steuerung auch veranlassen, dass für innerhalb des Zeitfensters neu erfasste Drucksignale das beschriebene Tropfenzählverfahren nicht durchgeführt wird, d.h. dass insbesondere keine Differenzbildung zwischen dem Signalwert und dem Basisdruckwert und auch kein Schwellenwertvergleich durchgeführt wird. Alternativ kann statt eines Zeitfensters eine Anzahl von Drucksignalen vorgegeben werden, die nicht zur Bestimmung der Tropfenzahl herangezogen werden sollen.

Gegenstand der Erfindung ist weiterhin ein Verfahren zur Bestimmung einer Konzentration eines Analyten, insbesondere eines oxidierbaren Inhaltsstoffes in einer flüssigen Probe, bei dem das voranstehend beschriebene Verfahren zum Bestimmen einer in ein Reaktionsgefäß eindosierten Tropfenzahl einen oder mehrere der Verfahrensschritte bildet. Dieses Verfahren zur Bestimmung einer Konzentration eines Analyten, insbesondere eines oxidierbaren Inhaltsstoffes, in einer flüssigen Probe, umfasst die Schritte:
- Betreiben einer Dosiereinrichtung, insbesondere einer Pumpe, um eine definierte Flüssigkeitsmenge tropfenweise mit einer Tropffrequenz über einen Flüssigkeitseinlass in ein Reaktionsgefäß einer Analysevorrichtung, welche insbesondere eine Hochtemperaturaufschlusseinrichtung umfasst, einzudosieren,
   wobei das Reaktionsgefäß von einem Gasstrom, insbesondere mit konstantem Volumenstrom, durchströmt wird, und wobei im Reaktionsgefäß eine Temperatur herrscht, welche größer ist als die Siedetemperatur der Flüssigkeit,
   und wobei ein Tropfen, insbesondere durch Wärmeübertragung in Kontakt mit einer Oberfläche innerhalb des Reaktionsgefäßes, unmittelbar nach dem Eintritt in das Reaktionsgefäß, insbesondere unmittelbar nach Kontakt mit der Oberfläche innerhalb des Reaktionsgefäßes, in die Gasphase übergeht;
- Bestimmen der Anzahl von in das Reaktionsgefäß eindosierten Tropfen der flüssigen Probe nach dem voranstehend beschriebenen Verfahren;
- daraus Ermitteln der eindosierten Flüssigkeitsmenge;
- Erfassen einer mit der Menge des Analyten korrelierten Messgröße; und
- Ermitteln der Konzentration des Analyten aus der Messgröße und der eindosierten Flüssigkeitsmenge.

Das mit der Menge das Analyten korrelierte Messsignal kann beispielsweise ein Signal eines optischen Detektors, wie des eingangs erwähnten Infrarot- oder Chemilumineszenz-Detektors sein.

Zum Ermitteln der eindosierten Probenmenge kann beispielsweise die Anzahl der in das Reaktionsgefäß eindosierten Tropfen der flüssigen Probe mit einem Referenzwert verglichen werden. Es ist auch möglich, aus der Anzahl der in das Reaktionsgefäß eindosierten Tropfen mit Hilfe eines beispielsweise durch vorherige Referenzmessungen bekannten Tropfenvolumens das Gesamtvolumen der eindosierten Flüssigkeit zu berechnen.

Der Referenzwert kann in einer Referenzmessung ermittelt werden, indem eine definierte Referenz-Flüssigkeitsmenge tropfenweise in das Reaktionsgefäß eindosiert wird, und die Anzahl der zum vollständigen Eindosieren der Referenz-Flüssigkeitsmenge notwendigen Tropfen als Referenzwert erfasst wird. Die Referenzmessung wird vorzugsweise unmittelbar nach Inbetriebnahme oder nach einer Wartungsmaßnahme an der Analysevorrichtung durchgeführt, wie beispielsweise nach dem Reinigen oder Ersetzen von Komponenten der Analysevorrichtung, da der ermittelte Referenzwert dann die Anzahl der unter Idealbedingungen zum vollständigen Eindosieren der Referenz-Flüssigkeitsmenge notwendigen Tropfen widerspiegelt.

Bei einer Abweichung der aktuell ermittelten Tropfenzahl von dem Referenzwert um mehr als einen vordefinierten Schwellenwert kann ein Alarm ausgegeben werden. Dabei wird der Schwellenwert vorzugsweise so definiert, dass bei einer Schwellenwertüberschreitung, d.h. einer größeren Abweichung der tatsächlich ermittelten Tropfenzahl von dem Referenzwert, das Analyseverfahren derart stark beeinträchtigt wird, dass auch mittels Korrekturrechnungen keine verlässliche Aussage über die gesuchte Analytkonzentration gemacht werden kann. Das Ausgeben des Alarms kann beispielsweise Wartungsmaßnahmen wie Reinigung des Flüssigkeitszulaufs oder das Ersetzen von Komponenten der Analysevorrichtung auslösen.

Bei einer Abweichung der aktuell ermittelten Tropfenzahl von dem Referenzwert um weniger als einen vordefinierten Schwellenwert, beispielsweise bei einer Abweichung von nur einem oder wenigen Tropfen, kann das Analyseergebnis mit einem Korrekturfaktor beaufschlagt werden. Beispielsweise kann in den Korrekturfaktor der Quotient aus der ermittelten Tropfenzahl und dem Referenzwert eingehen. Auf diese Weise geht die tatsächlich in das Reaktionsgefäß eindosierte Flüssigkeitsmenge in die Berechnung der Analytkonzentration ein, so dass auf einer falschen Probenmenge basierende Fehler bei der Konzentrationsberechnung vermieden werden.

Die Erfindung umfasst weiterhin eine Analysevorrichtung zur Bestimmung einer Konzentration eines Analyten, insbesondere eines oxidierbaren Inhaltsstoffes, in einer Flüssigkeitsprobe, welche eine Dosiereinrichtung, insbesondere eine Pumpe umfassend, zum tropfenweisen Eindosieren der Flüssigkeitsprobe in eine Hochtemperaturaufschlusseinrichtung zum Aufschließen der Flüssigkeitsprobe und Bildung eines Gasgemisches umfasst,
wobei die Hochtemperaturaufschlusseinrichtung ein Reaktionsgefäß mit einem Flüssigkeitseinlass für die Flüssigkeitsprobe, und eine Gaszuführung zur Zuführung eines Trägergases aufweist, und
über einen Gasauslass mit einer Analysekammer verbunden ist,
wobei sich im Betrieb der Vorrichtung ein Gasstrom eines Trägergases zwischen dem Gaseinlass und der Analysekammer ausbildet,
wobei in Richtung des Gasstroms vor der Analysekammer, insbesondere innerhalb der Gaszuführung zur Zuführung des Trägergases in das Reaktionsgefäß, ein Druckmesswandler angeordnet ist,
wobei der Druckmesswandler mit einer Kontrolleinheit zur weiteren Verarbeitung der vom Druckmesswandler ausgegebenen Drucksignale gekoppelt ist,
wobei die Kontrolleinheit Mittel zur Durchführung des voranstehend beschriebenen Verfahrens zur Bestimmung der in das Reaktionsgefäß eindosierten Tropfenzahl umfasst.

Die Kontrolleinheit kann eine zentrale Kontrolleinheit sein, die sämtliche Funktionen der Analysevorrichtung steuert und insbesondere das voranstehend beschriebene Verfahren zur Bestimmung der Konzentration eines Analyten in einer Flüssigkeitsprobe steuert bzw. durchführt. Dabei steuert die zentrale Kontrolleinheit insbesondere die Dosiereinrichtung zur tropfenweisen Zugabe der Flüssigkeitsprobe und wertet die Signale der in der Analysekammer angeordneten Detektionsvorrichtung aus. Insbesondere berechnet die zentrale Kontrolleinheit das Analyseergebnis und gibt dieses aus. Sie kann aber auch als separate Kontrolleinheit ausgestaltet sein.

Zwischen dem Gasauslass des Reaktionsgefäßes und der Analysekammer kann im Strömungsweg des Gasstroms eine Filtereinheit zum Entfernen von Feststoffpartikeln aus dem Gasstrom sowie eine Kondensationseinheit zum Auskondensieren von Wasser aus dem Gasstrom angeordnet sein.

Die Kontrolleinheit umfasst beispielsweise
eine Mittelungseinheit, die dazu ausgestaltet ist, aus der von dem Druckmesswandler empfangenen Folge von Drucksignalen einen Basisdruckwert, beispielsweise durch Bildung eines zeitlichen Mittelwerts oder eines gleitenden Mittelwerts, zu ermitteln;
einen Subtrahierer, der dazu ausgestaltet ist, eine Druckänderung, beispielsweise als Differenzsignal, aus einem von dem Druckmesswandler empfangenen aktuellen Drucksignal und dem Basisdruckwert zu bilden und auszugeben;
einen Schwellenwertdetektor, der dazu ausgestaltet ist, die Druckänderung mit einem vorgegebenen Schwellenwert zu vergleichen, und bei Schwellenwertüberschreitung ein Signal an einen mit dem Schwellenwertdetektor verbundenen Zähler auszugeben, der dazu ausgelegt ist bei Empfang des Signals einen hinterlegten Wert um eins zu erhöhen.

Der Zähler kann eine Reset-Funktion umfassen, die es erlaubt, den hinterlegten Wert, der die in das Reaktionsgefäß eindosierte Tropfenzahl repräsentiert, nach dem Eindosieren der gesamten Flüssigkeitsprobe auf Null zurückzusetzen.

Die Kontrolleinheit kann weiterhin einen Speicher umfassen, in dem beispielsweise ein Referenzwert hinterlegt werden kann, der eine bei einer Referenzmessung, bei der eine bekannte Menge einer Referenzflüssigkeit tropfenweise in das Reaktionsgefäß eindosiert wird, ermittelte Tropfenzahl repräsentiert.

Alle hier und im Folgenden beschriebenen Mittel der Kontrolleinheit zur Durchführung des Verfahrens zum Bestimmen einer Anzahl von Tropfen sind vorzugsweise als Software eines Mikroprozessors realisiert. Sie können jedoch mindestens teilweise auch als elektronische Schaltung realisiert sein.

Die Erfindung wird nun anhand der in der Zeichnung dargestellten Ausführungsbeispiele näher erläutert. Es zeigen:
- Fig. 1: eine schematische Darstellung einer Analysevorrichtung zur Analyse von flüssigen Proben;
- Fig. 2: einen Druckverlauf innerhalb des Reaktionsgefäßes der in Fig. 1 gezeigten Analysevorrichtung beim Eindosieren eines einzelnen Tropfens einer flüssigen Probe;
- Fig. 3: einen Druckverlauf innerhalb des Reaktionsgefäßes der in Fig. 1 gezeigten Analysevorrichtung beim Eindosieren einer Vielzahl von Tropfen einer flüssigen Probe und die Detektion der einzelnen Tropfen;
- Fig. 4: eine schematische Darstellung einer Kontrolleinheit zur Bestimmung der Anzahl der in ein Reaktionsgefäß eindosierten Tropfen.

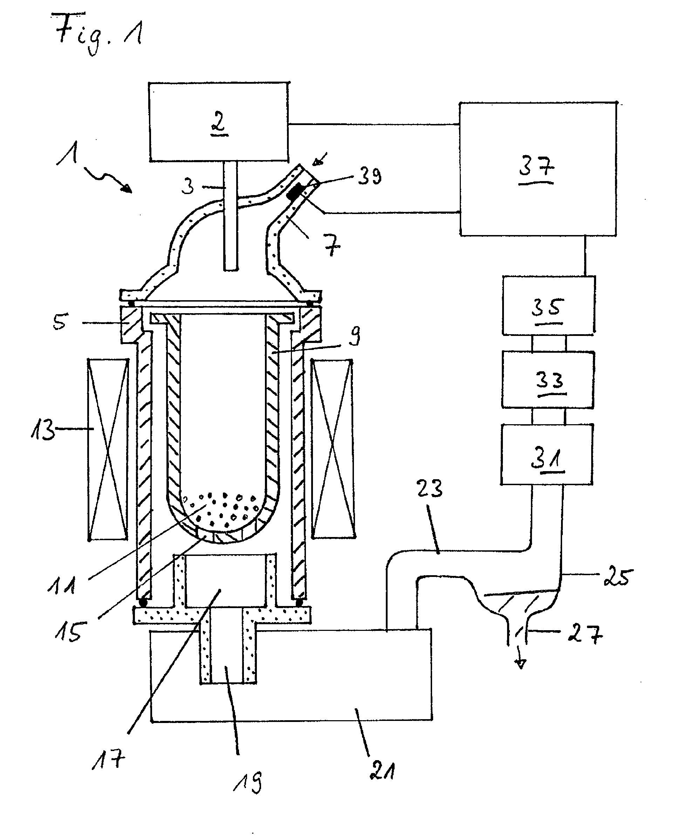

Bei der in Fig. 1 dargestellten Analysevorrichtung 1 für die Bestimmung beispielsweise des TOC- oder TN_{b}-Gehalts einer flüssigen Probe, beispielsweise einer Abwasserprobe, wird diese von einer nur schematisch dargestellten Dosiereinrichtung 2 über eine Einspritzdüse 3 einem beispielsweise als Pyrolyserohr ausgeführten Reaktionsgefäß 5 zugeführt. Gleichzeitig wird dem Reaktionsgefäß 5 über eine weitere Zuführung 7 ein sauerstoffhaltiges Trägergas zugeführt. Das Reaktionsgefäß 5 enthält im hier gezeigten Beispiel einen Einsatz 9, welcher einen Katalysator 11 enthält, der die Umsetzung der Flüssigkeitsprobe mit dem sauerstoffhaltigen Trägergas unterstützt. Um die Reaktion der Flüssigkeitsprobe mit dem Trägergas zu unterstützen, könnte gleichermaßen eine entsprechend hohe Innentemperatur des Reaktionsgefäßes 5 eingestellt werden. Die Temperatur des Reaktionsgefäßes 5 ist mittels einer das Reaktionsgefäß 5 umgebenden Heizvorrichtung 13 einstellbar. Im Bereich des Einsatzes 9 befindet sich die Reaktionszone, in der im Betrieb eine Temperatur zwischen 650 °C und 1300 °C herrscht. Optional kann innerhalb der Reaktionszone in dem Einsatz 9 Schüttgut (nicht eingezeichnet) aufgenommen sein, das durch den mit Durchtrittskanälen 15 versehenen Siebboden des Einsatzes 9 zurückgehalten wird. Im Kontakt einer Oberfläche im Inneren des Reaktionsgefäßes, beispielsweise mit der Oberfläche des Katalysators 11 oder des Schüttguts, erhitzen sich die Tropfen der Flüssigkeitsprobe innerhalb kürzester Zeit, nämlich innerhalb weniger Zehntelsekunden, insbesondere innerhalb weniger als 0,4 s, auf die Siede- bzw. Reaktionstemperatur und werden in die Gasphase überführt.

Unterhalb des Einsatzes 9 ist innerhalb des Reaktionsgefäßes 5 eine weitere Kammer 17 angeordnet, in der im Betrieb bereits eine niedrigere Temperatur herrscht als in der Reaktionszone. Am der Einspritzdüse 3 entgegengesetzen unteren Ende des im Betrieb vertikal ausgerichteten Reaktionsgefäßes 5 befindet sich ein Gasauslass 19, der im Innern einer Filtereinheit 21 mündet, so dass ein im Reaktionsgefäß 5 erzeugtes Gasgemisch über die Durchtrittskanäle 15, die Kammer 17 und den Gasauslass 19 mit dem Trägergas in die Filtereinheit 21 strömen kann. Die Filtereinheit 21 ist mit einer Kondensationseinheit 25 über eine Gasleitung 23 verbunden. Die Kondensationseinheit 25 dient zur Abscheidung von Wasser aus dem Gasstrom und ist deshalb gegebenenfalls mit einem Kühler versehen, um die Kondensation aus dem Gasstrom zu beschleunigen. Das Kondensat wird über eine Leitung 27 aus der Analysevorrichtung 1 entfernt.

In Strömungsrichtung des Gasstroms hinter der Kondensationseinheit 25 sind eine optionale Trocknungseinheit 31, ein weiterer Filter 33 und eine Analysekammer 35 angeordnet. In der Analysekammer 35 wird der Gehalt an im Gasstrom enthaltenen Reaktionsprodukten des Analyten, beispielsweise an CO₂ und/oder NOₓ, bestimmt. In der Regel wird zur Bestimmung des CO₂-Gehalts eine Infrarot-Messeinrichtung, z.B. ein Infrarot-Detektor verwendet. Zur Bestimmung des NOₓ-Gehalts wird in der Regel ein Chemilumineszenz-Detektor eingesetzt. Die in der Analysekammer 35 erfassten Messsignale werden einer Kontrolleinheit 37 mit einem Rechner, beispielsweise einem Mikrocontroller oder Mikroprozessor, zugeführt, die anhand der Messsignale die Konzentration des Analyten in der in das Reaktionsgefäß 5 eindosierten Probe bestimmt. Die Kontrolleinheit 37 steuert darüber hinaus auch die Dosiereinrichtung 2 zum Eindosieren der Flüssigkeit in das Reaktionsgefäß 5.

Der gesamte Strömungsweg des Trägergases ist gegenüber der Umgebung abgedichtet, so dass kein Gas aus der Analysevorrichtung 1 austreten kann. Durch einen Gasauslass (nicht eingezeichnet) der Analysekammer 37 tritt der Gasstrom aus der Analysevorrichtung 1 aus. Das Trägergas kann alternativ auch in einem Kreislaufprozess der Analysevorrichtung 1 wieder über die Gaszuführung 7 zugeführt werden. Die dem Reaktionsgefäß 5 nachgelagerten Komponenten der Analysevorrichtung 1 setzen dem Gasstrom einen Strömungswiderstand entgegen. Auf diese Weise wird es möglich, Druckänderungen im Inneren des Reaktionsgefäßes 5 auch in der Gaszuführung 7 zu detektieren, d.h. eine beispielsweise durch das Übergehen eines eindosierten Tropfens in die Gasphase bewirkte Druckänderung innerhalb des Reaktionsgefäßes 5 bewirkt eine damit korrelierte Druckänderung in der Gaszuführung 7. Ein in der Gaszuführung 7 angeordneter Druckmesswandler 39 erfasst den in der Gaszuführung 7 herrschenden Druck und wandelt diesen in ein von diesem Druck abhängiges, beispielsweise proportionales, elektrisches Signal, auch als Drucksignal bezeichnet, um. Aus einer Folge solcher Drucksignale lässt sich, wie weiter unten noch ausführlicher ausgeführt, auf Druckänderungen im Reaktionsgefäß 5 zurückschließen. Der Druckmesswandler 39 ist ausgangsseitig zur Übertragung von Drucksignalen mit einem Eingang der Kontrolleinheit 37 verbunden. Da der gesamte Strömungsweg des Trägergases gegenüber der Umgebung abgedichtet ist, kann der Druckmesswandler 39 zur Erfassung des innerhalb des Reaktionsgefäßes 5 herrschenden Drucks grundsätzlich an einer beliebigen Position entlang des Strömungsweges vorgesehen werden, beispielsweise im Bereich des Gasauslasses 19 oder innerhalb der Filtereinheit 21. Besonders vorteilhaft ist jedoch die Position innerhalb der Zuführung 7, da dort die Temperatur noch niedrig ist, beispielsweise im Bereich der Zimmertemperatur liegt.

Ein über die Einspritzdüse 3 in das Rekationsgefäß 5 eindosierter Tropfen einer flüssigen Probe geht nahezu unmittelbar nach dem Eintritt in die Rekationszone, insbesondere durch Wärmeübertragung im Kontakt mit einer heißen Oberfläche, in die Gasphase über. Handelt es sich bei der flüssigen Probe um eine wässrige Lösung, die neben Wasser auch oxidierbare Bestandteile enthält, geht beispielsweise das enthaltene Wasser durch Verdampfen in gasförmiges H₂O über, während oxidierbare Bestandteile, wie zum Beispiel organische kohlenstoff- oder stickstoffhaltige Verbindungen mit dem sauerstoffhaltigen Trägergas zu gasförmigen Oxiden wie CO₂ oder NOₓ reagieren. Dies macht sich durch einen Druckimpuls oder Druckstoß innerhalb des Reaktionsgefäßes 5 bemerkbar, der durch den in der Trägergaszuleitung 7 angeordneten Druckmesswandler 39 erfassbar ist.

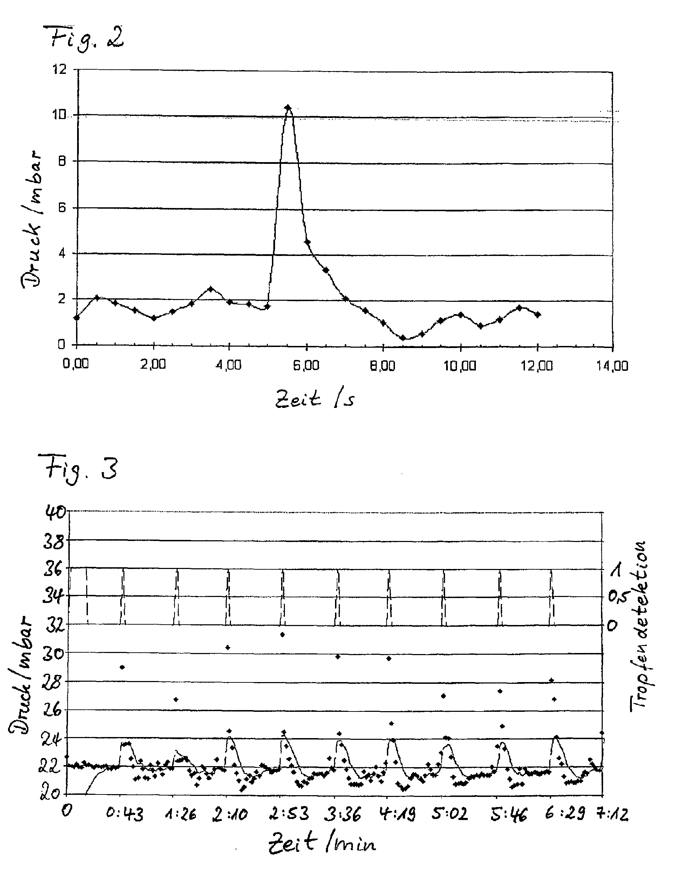

In Fig. 2 ist beispielhaft eine Folge von Druckmesswerten, die aus einer von dem Druckmesswandler 39 während des Eindosierens eines Flüssigkeitstropfens in das Reaktionsgefäß 5 ausgegebene Folge von Drucksignalen abgeleitet wurden, dargestellt. Auf der Abszisse des gezeigten Diagramms ist die Zeit in s dargestellt, auf der Ordinate der Druck in mbar. Die Rauten repräsentieren die einzelnen Messwerte der Folge. Wie aus dem Verlauf der Messwertfolge abzulesen ist, herrscht im Reaktionsgefäß im Zeitraum zwischen 0 und 5 s ein verhältnismäßig konstanter Druck zwischen 1 und knapp 3 mbar. Nach 5 s wird ein Tropfen in das Reaktionsgefäß eindosiert. Dieses Ereignis bewirkt eine starke Erhöhung des folgenden Druckmesswerts auf einen Wert von fast 11 mbar. Nach etwa 8 s ist der Druck wieder vollständig auf Werte im Bereich zwischen 0,5 und knapp 2 mbar abgeklungen.

Fig. 3 zeigt eine beim Eindosieren von neun Tropfen mit einer Tropffrequenz von ca. 8,6 Tropfen/min (d.h. alle 7 s wird ein neuer Tropfen eindosiert) in das Reaktionsgefäß 5 ermittelte Folge von Druckmesswerten. Auf der Abszisse des gezeigten Diagramms ist die Zeit in Minuten aufgetragen, auf der linken Ordinate der von dem Drucksensor 39 gemessene Druck in mbar. Die einzelnen aus den Drucksignalen des Druckmesswandlers 39 abgeleiteten Messwerte sind wiederum als Rauten dargestellt.

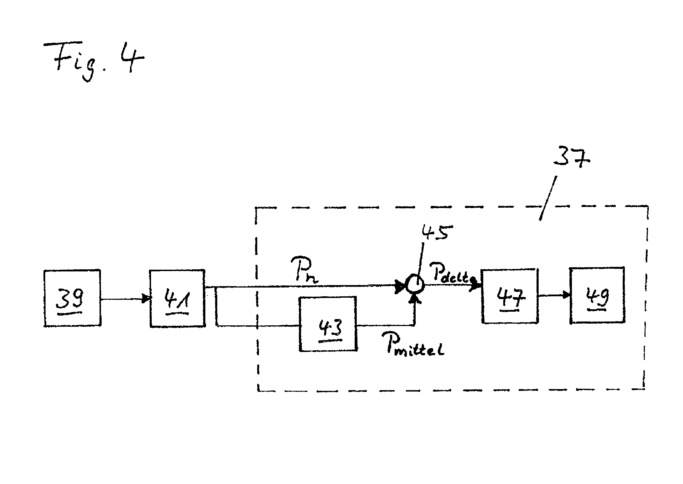

Die Auswertung der Folge von Drucksignalen des Druckmesswandlers 39 erfolgt mittels der mit dem Druckmesswandler 39 gekoppelten Kontrolleinheit 37 in der im folgenden beschriebenen Weise (vgl. Fig. 4): Die vom Druckmesswandler 39 gewandelten und gegebenenfalls von einem Verstärker 41 verstärkten Sensorsignale werden an die Kontrolleinheit 37, gegebenenfalls digitalisiert, weitergegeben. Das jeweils zuletzt erfasste Drucksignal Pₙ wird im Folgenden auch als aktuelles Drucksignal bezeichnet. Die Kontrolleinheit 37 umfasst eine Mittelungseinheit 43, welche einen zeitlichen Mittelwert zumindest über einige, dem aktuell erfassten Drucksignal Pₙ vorausgehende Drucksignale der Folge, beispielsweise als gleitenden Mittelwert über alle innerhalb eines vorgegebenen Zeitfensters erfassten Drucksignale, bildet. Gleichermaßen könnten statt einem Zeitfenster auch eine bestimmte Anzahl von, dem aktuellen Drucksignal in der Folge vorangehenden, Drucksignalen vorgegeben werden. Die Bildung des gleitenden Mittelwerts über mindestens einen Teil der dem aktuellen Drucksignal Pₙ vorausgehenden Drucksignale der Folge ist einem digitalen Tiefpassfilter vergleichbar. Entsprechend können auch andere vergleichbare Filterfunktionen eingesetzt werden. Der so erhaltene zeitliche Mittelwert bildet einen Basisdruckwert Pₘᵢₜₜₑₗ, welcher einem im Reaktionsgefäß 5 herrschenden Basisdruck entspricht. Der zeitliche Verlauf der Basisdruckwerte (gestrichelte Linie) bildet eine Art "Nulllinie" oder "Basislinie" des im Reaktionsgefäß 5 herrschenden Drucks. Druckimpulse aufgrund von in die Gasphase übergehenden Tropfen führen zu einer über diese Basislinie hinausgehende Zunahme des Drucks.

Die Basislinie verläuft im Idealfall im Wesentlichen parallel zur Abszisse des in Fig. 3 gezeigten Diagramms. Es ist jedoch möglich, dass die durch einen Tropfen verursachte Zunahme des Drucks innerhalb des Reaktionsgefäßes 5 nicht vollständig bis zum Eindosieren des nächsten Tropfens abgebaut wird. Ebenso ist es möglich, dass aufgrund von Verunreinigungen im Probengasstrom feste Partikel anfallen, die mit der Zeit die Filtereinheit 21 zusetzen. Beides führt zu einem allmählichen Anstieg des Basisdrucks im Reaktionsgefäß 5. Zur Überwachung der Analysevorrichtung 1 ist es deshalb möglich, mittels der Kontrolleinheit 37 den Basisdruckwert Pₘᵢₜₜₑₗ zu überwachen. Überschreitet der Basisdruckwert Pₘᵢₜₜₑₗ beispielsweise einen vorgegebenen Schwellenwert, kann dies ein Hinweis darauf sein, dass die Flüssigkeitsprobe zu schnell eindosiert wird oder dass eine zugesetzte Filtereinheit auszutauschen ist. In diesem Fall kann ein Alarm ausgegeben werden, der die Durchführung einer Wartungsmaßnahme auslöst.

Die Kontrolleinheit 37 umfasst weiterhin einen Subtrahierer 45, der eingangsseitig mit dem Druckmesswandler 39 und der Mittelungseinheit 43 gekoppelt ist. Der Subtrahierer bildet aus dem jeweils aktuellen Sensorsignal Pₙ und dem Basisdruckwert Pₘᵢₜₜₑₗ ein Differenzsignal, das einer Druckänderung P_{delta} zwischen dem aktuell erfassten Drucksignal Pₙ und dem Basisdruckwert Pₘᵢₜₜₑₗ entspricht.

Ein Schwellenwertdetektor 47 ist mit einem Ausgang des Subtrahierers 45 eingangsseitig gekoppelt, so dass das Differenzsignal P_{delta} vom Subtrahierer 45 an den Schwellenwertdetektor 47 übertragen werden kann. Der Schwellenwertdetektor 47 vergleicht das Differenzsignal P_{delta} mit einem vorgebbaren Schwellenwert. Der Schwellenwertdetektor 47 ist ausgangsseitig mit einem Zähler 49 verbunden. Liegt P_{delta} oberhalb des vorgegebenen Schwellenwerts, wird dies als "Tropfenereignis" interpretiert. Der Schwellenwertdetektor 47 gibt entsprechend ein Signal an den Zähler 49 aus, das den im Zähler 49 hinterlegten Zahlenwert um eins erhöht. Liegt P_{delta} dagegen unterhalb des Schwellenwerts, wird kein Signal an den Zähler 49 ausgegeben, so dass der dort hinterlegte Zahlenwert gleich bleibt. In Fig. 3 sind die Signale des Schwellenwertdetektors 47 als gestrichelte Signalspitzen (Peaks) markiert.

Da die zu den einzelnen Tropfen zugehörigen Druckimpulse erst über mehrere aufeinanderfolgende Drucksignale der Folge abklingen, besteht die Möglichkeit, dass pro Druckimpuls nicht nur ein einziges, sondern mehrere aufeinanderfolgende Drucksignale bzw. zugehörige Differenzsignale P_{delta} zwischen den aktuell erfassten Signalen und dem Basisdruckwert zu einer Schwellenwertüberschreitung führen. Um zu vermeiden, dass ein einzelner Impuls mehrfach gezählt wird, umfasst die Kontrolleinheit eine Funktion, die unmittelbar im Anschluss an eine Schwellwertüberschreitung den Schwellenwertdetektor 47 für ein vorgegebenes Zeitintervall, beispielsweise für die Länge einer typischen Abklingkurve, z.B. 2 s im Beispiel der Fig. 2, deaktiviert. Das Zeitintervall wird vorteilhafterweise mindestens so gewählt, dass die zum ersten, nach Ablauf des Zeitfensters erfassten aktuellen Drucksignal gehörige Druckänderung P_{delta} den vorgegebenen Schwellenwert sicher unterschreitet.

In Fig. 3 ist der Verlauf des Basisdruckwerts Pₘᵢₜₜₑₗ, der im vorliegenden Beispiel durch Bildung eines gleitenden Mittelwerts ermittelt wurde, als gestrichelte Linie dargestellt. Wenn die zu einem Druckimpuls gehörenden Drucksignale in den Basisdruckwert Pₘᵢₜₜₑₗ eingehen, ergibt sich entsprechend bei jedem neuen Druckimpuls ein Extremum des Basisdruckwerts Pₘᵢₜₜₑₗ. Es kann daher zweckmäßig sein, diejenigen Drucksignale, für die der Schwellenwertdetektor 47 eine Schwellenwertüberschreitung des Differenzsignals zwischen Basisdruckwert und Drucksignal feststellt, nicht in die Berechnung des Basisdruckwerts Pₘᵢₜₜₑₗ einzubeziehen. Auf diese Weise werden Druckimpulse noch sicherer als Schwellenwertüberschreitungen des Differenzsignals P_{delta} detektiert.

Im Folgenden wird ein Verfahren zu Bestimmung einer Konzentration eines Analyten in einer flüssigen Probe am Beispiel einer TOC-Bestimmung mit der in Fig. 1 dargestellten Analysevorrichtung 1 beschrieben.

Zunächst wird eine Referenzmessung durchgeführt, bei der unter idealen Bedingungen, beispielsweise unmittelbar nach der Wartung und Reinigung der Vorrichtung, eine bekannte Menge einer Referenzflüssigkeit, die hinsichtlich ihrer das Tropfenvolumen beeinflussenden Eigenschaften wie Viskosität, Dichte oder Oberflächenspannung im wesentlichen mit den im Analysebetrieb zu untersuchenden Flüssigkeitsproben übereinstimmt, tropfenweise in das Reaktionsgefäß 5 eindosiert. Falls es sich bei den Flüssigkeitsproben um Abwasserproben handelt, kommt als Referenzflüssigkeit beispielsweise eine mit einer Standardsubstanz aufgestockte Abwasserprobe in Frage.

Die Anzahl der zum Eindosieren der Referenzflüssigkeit notwendigen Tropfen wird beispielsweise mittels des im Zusammenhang mit Fig. 3 und 4 beschriebenen Zählverfahrens ermittelt. Dieser Wert dient für spätere Messungen als Referenzwert und kann in einem Speicher (nicht eingezeichnet) der Kontrolleinheit 37 hinterlegt werden.

Im Analysebetrieb löst die Kontrolleinheit 37 das tropfenweise Eindosieren einer Flüssigkeitsprobe in das Reaktionsgefäß 5 aus. Hierzu gibt sie einen entsprechenden Befehl an die Dosiereinrichtung 2 aus. Gleichzeitig setzt die Kontrolleinheit 37 den Zähler 49 mittels einer Reset-Funktion auf den Wert Null und aktiviert den Schwellenwertdetektor 47. Unmittelbar vor dem Beginn des Eindosierens der Flüssigkeitsprobe wird der Basisdruckwert Pₘᵢₜₜₑₗ auf einen Wert gesetzt, der den vor Beginn des Eindosierens der Probe innerhalb des Reaktionsgefäßes herrschenden Druck repräsentiert.

Nach Beginn des Eindosierens wird das zuvor beschriebene Zählverfahren zur Bestimmung der eindosierten Anzahl von Tropfen durchgeführt. Mit dem Trägergasstrom gelangen die Oxidationsprodukte des Analyten, im vorliegenden Beispiel CO₂, in die Analysekammer 35, die einen Detektor umfasst, hier einen Infrarotdetektor, der ein vom CO₂-Gehalt des Gasstroms abhängiges Signal an die Kontrolleinheit 37 ausgibt. Die Kontrolleinheit 37 ermittelt aus dem Signal des Infrarot-Detektors die im Gasstrom enthaltene CO₂-Menge, und daraus das Analyseergebnis, hier den CO₂-Gehalt.

Nachdem die Kontrolleinheit 37 die Eindosierung der Flüssigkeitsprobe beendet hat, wird auch das Zählverfahren beendet. Der im Zähler 49 zu diesem Zeitpunkt hinterlegte Wert wird mit dem aus der Referenzmessung gewonnenen Referenzwert verglichen. Wird dabei keine Abweichung zwischen dem Referenzwert und der ermittelten Tropfenzahl festgestellt, so wird der von der Kontrolleinheit 37 ermittelte CO₂-Gehalt als Analyseergebnis ausgegeben.

Wird dagegen eine große Abweichung, beispielsweise von mehr als 50%, zwischen dem Referenzwert und der ermittelten tatsächlich in das Reaktionsgefäß 5 eindosierten Tropfenzahl festgestellt, so wird ein Alarm ausgegeben. Dieser Alarm kann beispielsweise dazu dienen, eine Wartungsmaßnahme auszulösen, beispielsweise eine Reinigung der Analysevorrichtung 1 oder der Austausch von Komponenten der Analysevorrichtung 1.

Wird nur eine geringe Abweichung zwischen dem Referenzwert und dem im Zähler 49 hinterlegten Wert festgestellt, beispielsweise von wenigen Tropfen, so kann der von der Kontrolleinheit 37 anhand des Signals des Infrarot-Detektors ermittelte CO₂-Gehalt mit einem Korrekturfaktor beaufschlagt, und der korrigierte Wert als Analyseergebnis ausgegeben werden. Der Korrekturfaktor bewirkt, dass der tatsächlich im Gasvolumenstrom vorliegende Volumenanteil der Probe in die Bestimmung des im Gasstrom vorliegenden Analytgehalt eingeht. Der Korrekturfaktor kann beispielsweise im einfachsten Fall als Quotient aus dem Referenzwert und der tatsächlich eindosierten Tropfenzahl gebildet werden.

## Patentansprüche

1. Verfahren zum Bestimmen einer Anzahl von Tropfen, welche mit einer Tropffrequenz in ein Reaktionsgefäß (5), insbesondere in einer Hochtemperaturaufschlusseinrichtung für Analysatoren, eindosiert werden,
wobei das Reaktionsgefäß (5) von einem Gasstrom durchströmt wird,
und wobei im Reaktionsgefäß (5) eine Temperatur herrscht, welche größer ist als die Siedetemperatur der Flüssigkeit,
und wobei ein in das Reaktionsgefäß (5) eindosierter Tropfen, insbesondere durch Wärmeübertragung in Kontakt mit einer Oberfläche innerhalb des Reaktionsgefäßes (5), nach dem Eintritt in das Reaktionsgefäß, insbesondere unmittelbar nach Kontakt mit der Oberfläche innerhalb des Reaktionsgefäßes (5), mindestens teilweise in die Gasphase übergeht, **dadurch gekennzeichnet, dass** mit einer Abtastrate, welche größer ist als die Tropffrequenz, eine Folge von vom Druck innerhalb des Reaktionsgefäßes abhängigen Drucksignalen (Pₙ) erfasst wird, und aus der Folge von Drucksignalen oder daraus abgeleiteten Werten eine Anzahl von in das Reaktionsgefäß (5) eindosierten Tropfen ermittelt wird.

2. Verfahren nach Anspruch 1,
wobei zur Ermittlung der Anzahl der in das Reaktionsgefäß (5) eindosierten Tropfen aus der Folge von Drucksignalen ein aktuelles Drucksignal (Pₙ) durch Vergleich mit einem Basisdruckwert (Pₘᵢₜₜₑₗ) eine zum aktuellen Drucksignal (Pₙ) zugehörige Druckänderung (P_{delta}) ermittelt wird, und die Druckänderung (P_{delta}) mit einem vorgegebenen Schwellenwert verglichen wird, und auf Grundlage eines Ergebnisses des Vergleichs erfasst wird, ob die Druckänderung (P_{delta}) einem durch Eindosieren eines Tropfens in das Reaktionsgefäß (5) bewirkten Druckstoß entspricht.

3. Verfahren nach Anspruch 2,
wobei der Basisdruckwert (Pₘᵢₜₜₑₗ) unter Verwendung von mindestens zwei in der Folge von Drucksignalen dem aktuellen Drucksignal (Pₙ) vorangehenden Drucksignalen durch Mittelwertbildung, insbesondere durch gleitende Mittelwertbildung, gebildet wird.

4. Verfahren nach einem der Ansprüche 2 oder 3,
wobei das Eindosieren eines Tropfens in das Reaktionsgefäß (5) registriert wird, wenn die Druckänderung (P_{delta}) den vorgegebenen Schwellenwert überschreitet, und wobei zur Registrierung des Eindosierens eines Tropfens eine hinterlegte Anzahl von in das Reaktionsgefäß (5) eindosierten Tropfen um eins erhöht wird.

5. Verfahren nach einem der Ansprüche 2 bis 4,
wobei bei Überschreiten des vorgegebenen Schwellenwerts das zu der Druckänderung (P_{delta}) gehörige Drucksignal (Pₙ) nicht zur Berechnung des Basisdruckwerts (Pₘᵢₜₜₑₗ) verwendet wird.

6. Verfahren nach einem der Ansprüche 2 bis 5,
wobei bei Überschreiten des vorgegebenen Schwellenwerts auf das aktuelle Drucksignal (Pₙ) folgende, innerhalb eines vorgegebenen Zeitfensters erfasste Drucksignale bei der Bestimmung der Tropfenzahl nicht zu einer Registrierung des Eindosierens eines Tropfens führen, wobei insbesondere das besagte Zeitfenster so breit gewählt wird, dass die zum ersten, nach Ablauf des Zeitfensters erfassten Drucksignal gehörige Druckänderung den vorgegebenen Schwellenwert unterschreitet.

7. Verfahren zur Bestimmung einer Konzentration eines Analyten, insbesondere eines oxidierbaren Inhaltsstoffes, in einer flüssigen Probe, umfassend die Schritte:
- Betreiben einer Dosiereinrichtung (2), insbesondere einer Pumpe, um eine definierte Flüssigkeitsmenge tropfenweise mit einer Tropffrequenz über einen Flüssigkeitseinlass (3) in ein Reaktionsgefäß (5) einer Analysevorrichtung (1), welche insbesondere eine Hochtemperaturaufschlusseinrichtung umfasst, einzudosieren,
wobei das Reaktionsgefäß (5) von einem Gasstrom durchströmt wird, und wobei im Reaktionsgefäß (5) eine Temperatur herrscht, welche größer ist als die Siedetemperatur der Flüssigkeit;
- Bestimmen der Anzahl von in das Reaktionsgefäß (5) eindosierten Tropfen der flüssigen Probe nach dem Verfahren nach einem der Ansprüche 1 bis 6;
- daraus Ermitteln der eindosierten Flüssigkeitsmenge;
- Erfassen einer mit der Menge des Analyten korrelierten Messgröße; und
- Ermitteln der Konzentration des Analyten unter Verwendung der Messgröße und der eindosierten Flüssigkeitsmenge.

8. Verfahren nach Anspruch 7,
wobei zum Ermitteln der eindosierten Flüssigkeitsmenge die Anzahl der in das Reaktionsgefäß (5) eindosierten Tropfen der flüssigen Probe mit einem Referenzwert verglichen wird.

9. Verfahren nach Anspruch 8,
wobei der Referenzwert in einer Referenzmessung ermittelt wird, indem eine definierte Referenz-Flüssigkeitsmenge tropfenweise in das Reaktionsgefäß (5) eindosiert wird, und die Anzahl der zum vollständigen Eindosieren der Referenz-Flüssigkeitsmenge notwendigen Tropfen als Referenzwert erfasst wird, wobei die Referenzmessung insbesondere unmittelbar nach Inbetriebnahme oder einer Wartungsmaßnahme an der Analysevorrichtung (1), wie beispielsweise nach dem Reinigen oder Ersetzen von Komponenten der Analysevorrichtung (1), durchgeführt wird.

10. Verfahren nach einem der Ansprüche 8 oder 9,
wobei bei einer Abweichung der aktuell ermittelten Tropfenzahl von dem Referenzwert um mehr als einen vordefinierten Schwellenwert ein Alarm ausgegeben wird.

11. Verfahren nach einem der Ansprüche 8 oder 9,
wobei bei einer Abweichung der aktuell ermittelten Tropfenzahl von dem Referenzwert um weniger als einen vordefinierten Schwellenwert die ermittelte Konzentration des Analyten mit einem Korrekturfaktor beaufschlagt wird.

12. Verfahren nach Anspruch 11,
wobei in den Korrekturfaktor der Quotient aus der ermittelten Tropfenzahl und dem Referenzwert eingeht.

13. Analysevorrichtung (1) zur Bestimmung einer Konzentration eines Analyten, insbesondere eines oxidierbaren Inhaltsstoffes, in einer Flüssigkeitsprobe,
welche eine Dosiereinrichtung (2), insbesondere umfassend eine Pumpe, zum tropfenweisen Eindosieren der Flüssigkeitsprobe in eine Hochtemperaturaufschlusseinrichtung zum Aufschließen der Flüssigkeitsprobe und Bildung eines Gasgemisches umfasst,
wobei die Hochtemperaturaufschlusseinrichtung ein Reaktionsgefäß (5) mit einem Flüssigkeitseinlass (2) für die Flüssigkeitsprobe, und eine Gaszuführung (3) zur Zuführung eines Trägergases aufweist, und
über einen Gasauslass (19) mit einer Analysekammer (35) verbunden ist,
wobei sich im Betrieb der Vorrichtung ein Gasstrom eines Trägergases zwischen dem Gaseinlass (3) und der Analysekammer (35) ausbildet,
**dadurch gekennzeichnet, dass** in Richtung des Gasstroms vor der Analysekammer (35), insbesondere innerhalb der Gaszuführung (3) zur Zuführung des Trägergases, ein Druckmesswandler (39) angeordnet ist,
wobei der Druckmesswandler (39) mit einer Kontrolleinheit (37) zur weiteren Verarbeitung der vom Druckmesswandler (39) ausgegebenen Drucksignale gekoppelt ist,
wobei die Kontrolleinheit (37) Mittel zur Durchführung des Verfahrens nach einem der Ansprüche 1 bis 6 umfasst.

14. Analysevorrichtung (1) nach Anspruch 13,
wobei besagte Mittel der Kontrolleinheit (37) umfassen:
eine Mittelungseinheit (43), die dazu ausgestaltet ist, aus der von dem Druckmesswandler (39) empfangenen Folge von Drucksignalen (Pₙ) einen Basisdruckwert (Pₘᵢₜₜₑₗ), beispielsweise durch Bildung eines zeitlichen Mittelwerts oder eines gleitenden Mittelwerts, zu ermitteln;
einen Subtrahierer (45), der dazu ausgestaltet ist, eine Druckänderung (P_{delta}), beispielsweise als Differenzsignal, aus einem von dem Druckmesswandler (39) empfangenen aktuellen Drucksignal und dem Basisdruckwert (Pₘᵢₜₜₑₗ) zu bilden und auszugeben;
einen Schwellenwertdetektor (47), der dazu ausgestaltet ist, die Druckänderung (P_{delta}) mit einem vorgegebenen Schwellenwert zu vergleichen, und bei Schwellenwertüberschreitung ein Signal an einen mit dem Schwellenwertdetektor (47) verbundenen Zähler (49) auszugeben, der dazu ausgelegt ist bei Empfang des Signals einen hinterlegten Wert um eins zu erhöhen.

## Claims

1. Process to determine the number of drops which are metered into a reaction vessel (5), particularly in a high-temperature digestion unit for analyzers, at a drip frequency,
wherein gas flows through the reaction vessel (5)
and wherein the temperature in the reaction vessel (5) is higher than the boiling temperature of the liquid,
and wherein - particularly through heat transfer in contact with a surface inside the reaction vessel (5) - a drop metered into the reaction vessel (5) at least partially transfers to the gas phase once it enters the reaction vessel (5), particularly directly after coming into contact with the surface inside the reaction vessel (5),
**characterized in that** a range of pressure signals (Pₙ), which are dependent on the pressure inside the reaction vessel, is recorded at a scan rate that is greater than the drip frequency,
and a number of drops which are metered into the reaction vessel (5) is determined from the range of pressure signals and values derived from this.

2. Process as claimed in Claim 1,
wherein, to determine the number of drops metered into the reaction vessel (5) from the range of pressure signals, a current pressure signal (Pₙ) is compared with a basic pressure value (P_{avg}) to determine a pressure change (P_{delta}) associated with the current pressure signal (Pₙ), and the pressure change (P_{delta}) is compared with a predefined threshold value, and - based on the result of the comparison process - it is determined whether the change in pressure (P_{delta}) corresponds to a pressure surge brought about by metering a drop into the reaction vessel (5).

3. Process as claimed in Claim 2,
wherein the basic pressure value (P_{avg}) is formed by averaging at least two of the pressure signals in the range of pressure signals which occur before the current pressure signal (Pₙ), in particular using a floating average curve.

4. Process as claimed in one of the Claims 2 or 3,
wherein the metering of a drop into the reaction vessel (5) is recorded if the pressure change (P_{delta}) exceeds the predefined threshold value, and wherein - to record the metering of a drop - a saved number of drops which have been metered into the reaction vessel (5) is increased by one.

5. Process as claimed in one of the Claims 2-4,
wherein, when the predefined threshold value is exceeded, the pressure signal (Pₙ) associated with the change in pressure (P_{delta}) is not used to calculate the basic pressure value (P_{avg}).

6. Process as claimed in one of the Claims 2-5,
wherein, when the predefined threshold value is exceeded, pressure signals which follow the current pressure signal (Pₙ), and are recorded within a predefined timeframe, do not cause the metering of a drop to be recorded, wherein in particular the duration of said timeframe is such that the change in pressure associated with the first pressure signal recorded once the timeframe expires falls below the predefined threshold value.

7. Process to determine the concentration of an analyte, in particular an oxidizable substance, in a liquid sample, comprising the following steps:
- operation of a dosing unit (2), particularly a pump, to meter a defined quantity of liquid via a liquid inlet (3) into a reaction vessel (5) of an analytical unit (1) drop-by-drop at a drip frequency, said analytical unit in particular comprising a high-temperature digestion unit, wherein gas flows through the reaction vessel (5), and wherein the temperature in the reaction vessel (5) is higher than the boiling point of the liquid;
- determination of the number of drops of liquid sample metered into the reaction vessel (5) according to the process claimed in one of the Claims 1-6;
- using this information to determine the quantity of liquid metered;
- recording of a measured variable that correlates to the quantity of the analyte; and
- determination of the concentration of the analyte using the measured variable and the volume of liquid metered.

8. Process as claimed in Claim 7,
wherein to determine the amount of liquid metered, the number of drops of the liquid sample metered into the reaction vessel (5) is compared to a reference value.

9. Process as claimed in Claim 8,
wherein the reference value is determined in a reference measurement by metering a defined reference liquid volume into the reaction vessel (5) drop by drop, and recording - as the reference value - the number of drops required to fully meter the volume of reference liquid, wherein the reference measurement is particularly performed directly after commissioning or a maintenance measure on the analytical unit (1), such as after cleaning or replacing components of the analytical unit (1).

10. Process as claimed in one of the Claims 8 or 9,
wherein when the number of drops currently determined deviates from the reference value by more than a predefined threshold value an alarm is signaled.

11. Process as claimed in one of the Claims 8 or 9,
wherein when the number of drops currently determined deviates from the reference value by less than a predefined threshold value the determined concentration of the analyte is subject to a correction factor.

12. Process as claimed in Claim 11,
wherein the ratio between the determined number of drops and the reference value is incorporated into the correction factor.

13. Analytical unit (1) for determining a concentration of an analyte, in particular an oxidizable substance, in a liquid sample,
which comprises a dosing unit (1), particularly comprising a pump, for the drop-by-drop metering of the liquid sample into a high-temperature digestion unit to digest the liquid sample and form a gas mixture,
wherein the high-temperature digestion unit has a reaction vessel (5) with a liquid inlet (2) for the liquid sample, and a gas supply (3) to supply a carrier gas, and
is connected to an analytical chamber (35) via a gas outlet (19),
wherein when the unit is in operation a flow of carrier gas forms between the gas inlet (3) and the analytical chamber (35),
**characterized in that** a pressure transducer (39) is arranged in the direction of gas flow upstream from the analytical chamber (35), particularly within the gas supply (3) to supply the carrier gas,
wherein the pressure transducer (39) is coupled with a control unit (37) for the further processing of the pressure signals emitted by the pressure transducer (39),
wherein the control unit (37) comprises resources to carry out the process as claimed in one of the Claims 1 to 6.

14. Analytical unit (1) as claimed in Claim 13,
wherein said resources of the control unit (37) comprise:
an averaging unit (43) which is designed to determine a basic pressure value (P_{avg}) from the range of pressure signals (Pₙ) received from the pressure transducer (39), for example by forming an average over time or a floating average;
a subtracting point (45) which is designed to form and output a pressure change (P_{delta}), for example as a differential signal, from a pressure signal currently received from the pressure transducer (39) and the basic pressure value (P_{avg});
a threshold value detector (47), which is designed to compare the pressure change (P_{delta}) with a predefined threshold value and, when the threshold value is exceeded, to output a signal to a counter (49) that is connected to the threshold detector (47), said counter being designed to increase a stored value by one when the signal is received.

## Revendications

1. Procédé destiné à la détermination d'un nombre de gouttes, lesquelles sont dosées avec une fréquence des gouttes dans un récipient de réaction (5), notamment dans une unité de digestion haute température pour analyseurs,
pour lequel le récipient de réaction (5) est parcouru par un flux de gaz,
et pour lequel il règne, dans le récipient de réaction (5), une température supérieure à la température d'ébullition du liquide,
et pour lequel une goutte dosée dans le récipient de réaction (5) se transforme au moins partiellement en phase gazeuse, notamment par la transmission de chaleur en contact avec une surface à l'intérieur du récipient de réaction (5), après l'entrée dans le récipient de réaction, notamment immédiatement après contact avec la surface à l'intérieur du récipient de réaction (5), **caractérisé en ce qu'**avec une vitesse d'échantillonnage, laquelle est supérieure à la fréquence des gouttes, une série de signaux de pression (Pₙ) dépendant de la pression à l'intérieur du récipient de réaction est saisie, et **en ce qu'**un nombre de gouttes dosées dans le récipient de réaction (5) est déterminé sur la base de la série de signaux de pression ou des valeurs en découlant.

2. Procédé selon la revendication 1,
pour lequel est déterminé, en vue de la détermination du nombre de gouttes dosées dans le récipient de réaction (5), un signal de pression actuel (Pₙ) à partir de la série de signaux de pression, par comparaison avec une valeur de pression de base (P_{moy.}), une variation de pression (P_{delta}) associée au signal de pression actuel (Pₙ), et pour lequel la variation de pression (P_{delta}) est comparée avec un seuil prédéfini, et pour lequel est déterminé, sur la base du résultat de la comparaison, si la variation de pression (P_{delta}) correspond à un à-coup de pression occasionné par le dosage d'une goutte dans le récipient de réaction (5).

3. Procédé selon la revendication 2,
pour lequel la valeur de pression de base (P_{moy.}) est formée en utilisant au moins deux signaux de pression dans la série de signaux de pression, précédant le signal de pression actuel (Pₙ), par la formation d'une moyenne, notamment par la formation d'une moyenne mobile.

4. Procédé selon l'une des revendications 2 ou 3,
pour lequel le dosage d'une goutte dans le récipient de réaction (5) est enregistré lorsque la variation de pression (P_{delta}) dépasse le seuil prédéfini, et pour lequel un nombre prédéfini de gouttes dosées dans le récipient de réaction (5) est incrémenté en vue de l'enregistrement du dosage d'une goutte.

5. Procédé selon l'une des revendications 2 à 4,
pour lequel, en cas de dépassement du seuil prédéfini, le signal de pression (Pₙ) associé à la variation de pression (P_{delta}) n'est pas utilisé pour le calcul de la valeur de pression de base (P_{moy.})

6. Procédé selon l'une des revendications 2 à 5,
pour lequel, en cas de dépassement du seuil prédéfini, les signaux de pression mesurés au sein d'une fenêtre de temps prédéfinie, consécutifs au signal de pression (Pₙ) actuel, ne donnent pas lieu à un enregistrement du dosage d'une goutte lors de la détermination du nombre de gouttes, pour lequel notamment ladite fenêtre de temps est choisie avec une largeur telle que la variation de pression associée au premier signal de pression, mesuré après l'écoulement de la fenêtre de temps, parvient sous le seuil prédéfini.

7. Procédé destiné à la détermination d'une concentration d'un analyte, notamment d'un ingrédient oxydable, dans un échantillon liquide, comprenant les étapes suivantes :
- Exploitation d'une unité de dosage (2), notamment d'une pompe, afin de doser goutte à goutte une quantité de liquide définie à une fréquence de gouttes, par l'intermédiaire d'une entrée de liquide (3) dans un récipient de réaction (5) d'un analyseur (1), lequel comprend notamment une unité de digestion haute température,
pour lequel le récipient de réaction (5) est parcouru par un flux gazeux, et pour lequel il règne à l'intérieur du récipient de réaction (5) une température supérieure à la température d'ébullition du liquide ;
- Détermination du nombre de gouttes de l'échantillon liquide dosées dans le récipient de réaction (5), d'après le procédé selon l'une des revendications 1 à 6 ;
- Déduction de la quantité de liquide dosée sur la base de la détermination précédente ;
- Mesure d'une grandeur corrélée avec la quantité de l'analyte ; et
- Détermination de la concentration de l'analyte en utilisant la grandeur mesurée et la quantité de liquide dosée.

8. Procédé selon la revendication 7,
pour lequel est comparée, pour la détermination de la quantité de liquide dosée, la goutte de l'échantillon liquide dosée dans le récipient de réaction (5) avec une valeur de référence.

9. Procédé selon la revendication 8,
pour lequel la valeur de référence est déterminée dans le cadre d'une mesure de référence, en ce qu'une quantité de liquide de référence est dosée goutte à goutte dans le récipient de réaction (5), et le nombre des gouttes nécessaires au dosage complet de la quantité de liquide de référence est mesuré en tant que valeur de référence, pour lequel la mesure de référence est réalisée notamment immédiatement après la mise en service ou une mesure de maintenance sur l'analyseur (1), comme par exemple après le nettoyage ou le remplacement de composants de l'analyseur (1).

10. Procédé selon l'une des revendications 8 ou 9,
pour lequel, en cas d'écart supérieur à un seuil prédéfini du nombre de gouttes actuellement déterminé par rapport à la valeur de référence, une alarme est émise.

11. Procédé selon l'une des revendications 8 ou 9,
pour lequel, en cas d'écart inférieur à un seuil prédéfini du nombre de gouttes actuellement déterminé par rapport à la valeur de référence, la concentration déterminée de l'analyte est soumise à un facteur de correction.

12. Procédé selon la revendication 11,
pour lequel le quotient entre le nombre de gouttes déterminé et la valeur de référence est pris en compte dans le facteur de correction.

13. Analyseur (1) destiné à la détermination d'une concentration d'un analyte, notamment d'un ingrédient oxydable, dans un échantillon liquide,
lequel comprend une unité de dosage (2), comprenant notamment une pompe destinée à doser goutte à goutte l'échantillon liquide dans une unité de digestion haute température destinée à la digestion de l'échantillon liquide et à la formation d'un mélange gazeux, pour lequel l'unité de digestion haute température comporte un récipient de réaction (5) doté d'une entrée de liquide (2) pour l'échantillon liquide, et une alimentation de gaz (3) destinée à l'alimentation d'un gaz porteur, et
est reliée avec une chambre d'analyse (35) par l'intermédiaire d'une sortie de gaz (19), pour lequel se forme, pendant le fonctionnement de l'analyseur, un flux de gaz porteur entre l'entrée de gaz (3) et la chambre d'analyse (35),
**caractérisé en ce qu'**en direction du flux de gaz en amont de la chambre d'analyse (35), notamment au sein de l'alimentation de gaz (3) destinée à l'alimentation du gaz porteur, est disposé un convertisseur de mesure de pression (39),
le convertisseur de mesure de pression (39) étant couplé avec une unité de contrôle (37) destinée au traitement aval des signaux de pression émis par le convertisseur de mesure de pression (39),
l'unité de contrôle (37) comprenant des moyens pour l'exécution du procédé selon l'une des revendications 1 à 6.

14. Analyseur (1) selon la revendication 13,
pour lequel lesdits moyens de l'unité de contrôle (37) comprennent :
une unité de formation de moyenne (43), laquelle est conçue en vue de déterminer, à partir de la série de signaux de pression (Pₙ) reçue par le convertisseur de mesure de pression (39), une valeur de pression de base (P_{moy.}), par exemple par la formation d'une moyenne temporelle ou d'une moyenne mobile ;
un soustracteur (45), lequel est conçu en vue de former et d'émettre une variation de pression (P_{delta}), par exemple en tant que signal différentiel, à partir d'un signal de pression actuel reçu par le convertisseur de mesure de pression (39) et de la valeur de pression de base (P_{moy.}) ;
un détecteur de seuil (47), lequel est conçu en vue de comparer la variation de pression (P_{delta}) avec un seuil prédéfini, et en cas de dépassement du seuil, en vue d'émettre un signal à un compteur (49) relié avec le détecteur de seuil (47), lequel compteur est conçu de telle sorte à incrémenter une valeur enregistrée à la réception du signal.
